Europäisches Patentamt

European Patent Office

Office européen des brevets

(11) Veröffentlichungsnummer: **0 132 557**
**B1**

(12) **EUROPÄISCHE PATENTSCHRIFT**

(45) Veröffentlichungstag der Patentschrift: **10.10.90**

(21) Anmeldenummer: **84106464.5**

(22) Anmeldetag: **06.06.84**

(51) Int. Cl.⁵: **C 12 P 7/58,** C 12 P 7/18, C 12 M 1/40 // C12P17/06

(54) **Verfahren zur kontinuierlichen enzymatischen Herstellung von Gluconsäure oder ihren Derivaten und Sorbit und/oder Mannit.**

(30) Priorität: **22.07.83 DE 3326546**

(43) Veröffentlichungstag der Anmeldung:
**13.02.85 Patentblatt 85/07**

(45) Bekanntmachung des Hinweises auf die Patenterteilung:
**10.10.90 Patentblatt 90/41**

(84) Benannte Vertragsstaaten:
**BE CH DE FR GB IT LI NL**

(56) Entgegenhaltungen:
**DD-A- 157 107**
**FR-A-2 398 046**

**ENZYME NOMENCLATURE, 1973, Elsevier Scientific Publishing Co., Amsterdam, NL, Seiten 44-51**

(73) Patentinhaber: **FRAUNHOFER-GESELLSCHAFT ZUR FÖRDERUNG DER ANGEWANDTEN FORSCHUNG E.V.**
**Leonrodstrasse 54**
**D-8000 München 19 (DE)**

(72) Erfinder: **Kulbe, Klaus Dieter, Dr. Dipl.-Chem.**
**Ritterstrasse 12**
**D-7031 Gärtringen 2 (DE)**
Erfinder: **Schwab, Ursula**
**Schwenkgasse 65**
**D-7317 Wendlingen (DE)**
Erfinder: **Chmiel, Horst, Prof. Dr.-Ing.**
**Paracelsiusstrasse 14**
**D-7250 Leonberg (DE)**
Erfinder: **Strahtmann, Heinrich, Dr.-Ing.**
**Milanweg 15**
**D-7400 Tübingen (DE)**

(74) Vertreter: **Kraus, Walter, Dr. et al**
**Patentanwälte Kraus, Weisert & Partner**
**Thomas-Wimmer-Ring 15**
**D-8000 München 22 (DE)**

# EP 0 132 557 B1

(56) Entgegenhaltungen:

ANALYTICAL LETTERS, Band 9, Nr. 3, 1976,
Seiten 257-276, Marcel Dekker, Inc.; W.H.
BARICOS et al.: "Practical processing with
cofactor-requiring enzymes key words:
cofactor-regeneration, immobilized cofactors,
enzymes"

CHEMICAL ABSTRACTS, Band 95, Nr. 17, 26.
Oktober 1981, Seite 297, Zusammenfassung Nr.
146615n, Columbus, Ohio, US

CHEMICAL ABSTRACTS, Band 90, Nr. 11, 12.
März 1979, Seite 446, Zusammenfassung Nr.
85255t, Columbus, Ohio, US; P.R. CHAMBERS et
al.: "High turnover NAD regeneration in the
coupled dehydrogenase conversion of sorbitol
to fructose"

**Beschreibung**

Die Erfindung betrifft ein Verfahren zur Herstellung von Gluconsäure und/oder ihren Derivaten und Sorbit oder Mannit oder Sorbit und Mannit aus Gemischen, die Glucose und Fructose enthalten oder aus Disacchariden, die Glucose und/oder Fructose enthalten.

Gluconsäure wird bisher industriell nur fermentativ hergestellt. Die anfänglich benutzten Drehtrommel- und Submersverfahren sind inzwischen durch ein Verfahren mit Aspergillus niger ersetzt worden, bei dem Gluconsäure, Gluconate und Gluconolacton sowie Glucoseoxidase aus den Mycelien gewonnen werden (vgl. US-Pat. 3.669.840). Bei diesen Verfahren zur Gluconsäuregewinnung stellt Sauerstoff den eigentlich limitierenden Faktor dar. Ausgangssubstrat ist Glucose bzw. Maltose. Versuche mit künstlichen Zellen (Hefezellen + Glucoseoxidase in Polyacrylamid) und immobilisierten Enzymen wurden an verschiedenen Stellen durchgeführt, blieben bisher jedoch ohne industrielle Anwendung, nicht zuletzt aufgrund der zu geringen Stabilität und Standzeit der Gesamtsysteme. Die mögliche chemische Synthese der Gluconsäure ist mit hohen Energiekosten verbunden und wie bei den meisten fermentativen Verfahren müssen auch hier die Produkte von großen Mengen an Begleitstoffen getrennt werden.

Gluconsäure findet auf vielen Gebieten Verwendung. Beispielsweise wird sie als milde Säure bei der Herstellung von Wurst- und Fleischwaren, Backpulver und Milchprodukten eingesetzt. Sie findet weiterhin Verwendung als Reinigungsmittel, als Mittel zur Metalloberflächenbehandlung und als Gerbemittel. Auch auf pharmazeutischem Gebiet wird sie eingesetzt. Als Ausgangsverbindung besitzt sie Bedeutung bei der Synthese von Ketogluconsäuren, für Vorstufen der Vitamin-C-Synthese (Ascorbinsäure) und für die Gewinnung von D-Arabinose innerhalb der Synthese von Riboflavin. Ihr Derivat, das Gluconsäure-δ-lacton findet Verwendung in Reinigungsmitteln zur Verhütung von Milchstein und Bierstein, als Katalysator bei der Herstellung saurer Kolloidharze, zur Herstellung von Wasserfarben und bei der Behandlung von Metalloberflächen.

Die Zuckeralkohole Sorbit und Mannit wurden bisher nur durch katalytische Reduktion von Invertzucker unter Verwendung von Nickelkatalysatoren hergestellt. Ihre Synthese ist sehr energieaufwendig und führt zu mehreren Nebenprodukten, so daß die Ausbeuten entsprechend vermindert sind und die Aufarbeitung der Reaktionsprodukte kompliziert abläuft. Gleiches gilt für die von Glucose bzw. Fructose ausgehenden reduktiven Verfahren (Natriumborhydrid, elektrolytisches Verfahren).

Beide Zuckeralkohole werden für diätetische Lebensmittel als Zuckeraustauschstoffe mit nur geringer kariogener Wirkung besonders für Diabetiker, als Feuchthaltemittel anstelle von Glycerin, Weichmacher, Kristallisationsverzögerer und zur Verbesserung der Rehydratation von Trockenprodukten, außerdem als Rohstoff für Lacke, Firnisse, Tenside und Explosivstoffe in der Papier-, Leder- und Textilindustrie und bei der Leim- und Gelatine-Herstellung verwendet. Sorbit ist eine Vorstufe für Sorbose, die ein wesentliches Zwischenprodukt der Vitamin-C-Synthese darstellt. Der Weltjahresbedarf an Sorbit und Mannit beträgt derzeit ca. 400.000 Tonnen.

Der Einsatz von Enzymmembranreaktoren stellt ein noch relativ junges Entwicklungsgebiet innerhalb der Enzymtechnologie dar. Obwohl einige wenige Prozesse wie z.B. die Herstellung von L-Aminosäuren aus α-Ketocarbonsäuren, (DE-OS 29 30 070) bereits technisch durchgeführt werden, sind Reaktionen mit Coenzymregenerationssystemen für industrielle Anwendung bisher noch nicht ausreichend untersucht worden. Verfahren, bei denen sowohl die Oxidationsreaktion als auch die Reduktionsreaktion wirtschaftlich interessante Produkte liefern, sind bisher nicht bekannt geworden. Die Herstellung polymergebundener Coenzyme wird in der DE-OS 28 41 414 beschrieben. Der Einsatz von Membrantrennverfahren zur Aufarbeitung der Produkte in biotechnologischen Prozessen steht ebenfalls erst am Anfang einer aussichtsreichen Entwicklung.

In der FR-A-2 398 046 wird ein enzymatisches Coenzymregenerationssystem zur nicht-kontinuierlichen Herstellung von Carnitin und Gluconolacton beschrieben, das als Ausgangssubstanzen 3-Hydrocarnitin und Glucose, als Enzyme Carnitin-Dehydrogenase und Glucose-Dehydrogenase und als eventuelles immobilisiertes Redoxsystem NAD$^+$/NADH enthält. Die Produkte Carnitin und Gluconolacton sind wirtschaftlich von Interesse. Prozesse mit Coenzymregeneration, auch kontinuierliche Prozesse, sind aus der Literaturstelle Anal. Lett. 9 (1976), 257 — 276 bekannt. In Enzyme Nomenclature 1973 (Elsevier), Seiten 44, 45, 48, 49, 50 und 51 werden allgemein bekannte Enzyme für die Zuckeralkohol-Synthese aus Fructose beschrieben.

Der vorliegenden Erfindung liegt die Aufgabe zugrunde, ein enzymatisches Verfahren zur Herstellung von Gluconsäure und/oder ihren Derivaten und Sorbit oder Mannit oder Sorbit und Mannit zur Verfügung zu stellen. Bei dem Verfahren soll eine Coenzymregeneration ablaufen. Bei dem Verfahren sollen als Ausgangsmaterialien leicht verfügbare Stoffe verwendet werden, und das Verfahren soll auf einfache Weise kontinuierlich ablaufen können.

Gegenstand der Erfindung ist ein Verfahren zur kontinuierlichen enzymatischen Herstellung von Gluconsäure und/oder ihren Derivaten und

i. Sorbit oder

ii. Mannit oder

iii. Sobit und Mannit

aus Glucose und/oder Fructose oder Glucose- und/oder Fructose enthaltenden Disacchariden, das dadurch gekennzeichnet ist, daß man das als Ausgangssubstrat vorliegende Disaccharid in einem mit dem

3

Redox-Vorgang gleichzeitig ablaufenden oder vorgeschalteten Reaktionsschritt durch spezifische Glykosidasen in die entsprechenden Monosaccharide spaltet und daß man gegebenenfalls ein Glucose-Fructose-Verhältnis von ca. 1:1 durch ebenfalls gleichzeitige oder vorgeschaltete enzymatische Isomerisierung mittels glucoseumsetzender Xylose-Isomerase oder durch chemische Isomerisierung einstellt und in einen Reaktor, der Glucose-Dehydrogenase und

i. Sorbit-Dehydrogenase oder

ii. Mannit-Dehydrogenase oder

iii. Polyol-Dehydrogenase sowie gegebenenfalls noch weitere Dehydrogenasen, Isomerasen oder spezifische Glykosidasen und ein

zusätzliches Redoxsystem enthält, kontinuierlich eine wäßrige Lösung von Glucose und/oder Fructose oder eine wäßrige Lösung von einem Glucose und/oder Fructose enthaltenden Disaccharid zuführt und aus dem Reaktor kontinuierlich eine wäßrige Lösung mit den gebildeten Produkten abführt.

Eine bevorzugte erfindungsgemäße Verfahrensform ist dadurch gekennzeichnet, daß man die Oxidations-Reduktionsreaktionen in einem Reaktor mit Ultrafiltrationsmembran durchführt, ein Coenzymsystem verwendet, das an ein wasserlösliches Polymer mit einem durchschnittlichen Molekulargewicht zwischen 5.000 und 60.000 Dalton gebunden ist, über die Membran eine Druckdifferenz erzeugt und hinter der Membran einen kontinuierlichen Produktstrom abführt.

Durch das erfindungsgemäße Verfahren ist es möglich, zwei industriell unterschiedliche Produktgruppen, nämlich die Gluconsäure und ihre Derivate sowie die Zuckeralkohole Mannit und Sorbit zu gewinnen. Damit kann der Zuckerindustrie und der Stärkeindustrie ein saisonunabhängiges Produktionsverfahren für zusätzliche und alternative Produkte zur Verfügung gestellt werden. Insbesondere ist das erfindungsgemäße Verfahren für die Verarbeitung der bei der Fructose-Produktion als Nebenprodukt anfallenden Glucose-Fructose-Gemische geeignet. Hierbei sind z.B. Durchsätze von ca. 1 Tonne/Stunde bei Feststoffgehalten von 15 — 20% üblich.

Mit dem erfindungsgemäßen enzymtechnologischen Verfahren zur Herstellung von Gluconsäure und ihrer Derivate steht eine Alternative zum fermentativen Verfahren zur Verfügung. Das neue Verfahren überwindet gleichzeitig die beiden grundsätzlichen Limitierungen des verschiedentlich erfolglos erprobten enzymatischen Verfahrens mit Glucose-Oxidase und Katalase (1. schlechte Löslichkeit des Substrats $O_2$ in Wasser und 2. die Hemmung beider Enzyme durch das Zwischenprodukt $H_2O_2$).

Außerdem wird mit dem erfindungsgemäßen Verfahren ein enzymtechnologischer Prozeß zur Herstellung von Sorbit bzw. Mannit zur Verfügung gestellt, der von der Stärke-Industrie sehr gewünscht, bisher jedoch wegen der mit der Coenzymregeneration verbundenen Probleme und Kosten nicht verwirklicht werden konnte. Mit der erfindungsgemäßen Einführung des beschriebenen Systems zur Coenzymregeneration wird gleichzeitig ein zweites industriell interessantes Produkt erzeugt. Der Gesamtprozeß kann damit ökonomischer gestaltet werden.

Es sind Enzyme bekannt, die bevorzugt den Hydrierungsvorgang (Reduktion) katalysieren, also zur Bildung von Polyhydroxyalkoholen (Zuckeralkoholen) aus den entsprechenden Zuckern führen. Diese Reaktionsrichtung wird durch kontinuierliche Abfuhr der gebildeten Zuckeralkohole aus der Reaktionsmischung mittels des erfindungsgemäßen Membranreaktors noch stärker bevorzugt.

Das erfindungsgemäße Verfahren wird anhand des folgenden Reaktionsschemas näher erläutert.

Bei dem erfindungsgemäßen Verfahren kann beispielsweise als Ausgangsmaterial ein Gemisch verwendet werden, das Glucose und Fructose enthält. Bei der Herstellung von Fructose aus Saccharose fällt in der Zuckerindustrie als Nebenprodukt ein Fructose-Glucose-Gemisch an. Dieses ist für das erfindungsgemäße Verfahren besonders geeignet. Das Glucose-Fructose-Gemisch kann Glucose und Fructose in beliebigen Anteilen enthalten, da Glucose und Fructose reversibel ineinander durch glucoseumsetzende Xylose-Isomerase umwandelbar sind.

Bei der Stärkehadrolyse (Amylose) erzeugter Stärkesirup besteht aus einem Gemisch von Glucose, Maltose und Dextrinen.

$$\text{Inulin} \xrightarrow{\quad 10 \quad} \text{n-Fructose}$$

Saccharose $\xrightarrow{\;6\;}$ Glucose+Fructose

Maltose $\xrightarrow{\;7\;}$ 2xGlucose

Cellobiose $\xrightarrow{\;8\;}$ 2xGlucose

Lactose $\xrightarrow{\;9\;}$ Glucose+Galactose

$$\text{Glucose} \xrightleftharpoons{\quad 5 \quad} \text{Fructose}$$

Glucose $\xrightarrow{\;1\;}$ Gluconsäure

NAD$^+$     NADH

Sorbit $\xleftarrow{\;2\;}$ Fructose

Mannit $\xleftarrow{\;3\;}$ Fructose

Sorbit + Mannit $\xleftarrow{\;4\;}$ Fructose

1. Glucose - Dehydrogenase
2. Sorbit - Dehydrogenase
3. Mannit - Dehydrogenase
7. Maltase (Glucoamylase)
9. β -Galactosidase

4. Polyol - Dehydrogenase
5. Xylose - Isomerase
6. β -Fructofuranosidase (Invertase)
8. Cellobiase
9. Inulinase

Dieses Gemisch kann nach weiterer enzymatischer (Glucoamylase, Glucoseisomerase) oder chemischer Umsetzung ebenfalls als Ausgangssubstrat für die kontinuierliche Herstellung von Gluconsäure und Sorbit bzw. Mannit mit dem erfindungsgemäßen Verfahren eingesetzt werden.

Als Substrate für das erfindungsgemäße Verfahren können außerdem Disaccharide, die Glucose und/ oder Fructose enthalten, eingesetzt werden. Beispiele für solche Disaccharide sind Saccharose, Cellobiose, Maltose oder Lactose. Die Disaccharide werden so ausgewählt, daß sie durch chemische oder enzymatische Spaltung und gegebenenfalls durch eine nachfolgende Isomerisierung leicht in Glucose-Fructose-Gemische überführbar sind. Es ist ein besonderer Vorteil des erfindungsgemäßen Verfahrens, daß man als Substrat Lactose verwenden kann. Lactose fällt in beachtlichen Mengen in der Milchindustrie (Molke) an und die Beseitigung der Lactose ist derzeit eine große Schwierigkeit, da bei der Milchindustrie große Abwassermengen anfallen, die Lactose enthalten.

Verwendet man bei dem erfindungsgemäßen Verfahren als Ausgangsmaterial ein Gemisch aus Glucose und Fructose, so enthält dieses die beiden Bestandteile bevorzugt in einem Verhältnis von 1:1.

Verwendet man als Ausgangsmaterialien reine Glucose oder Disaccharide, so wird die Spaltung der Disaccharide und die Isomerisierung der Glucose bevorzugt so eingestellt, daß Gemische, die Glucose und Fructose in einem Mengenverhältnis 1:1 enthalten, resultieren.

Erfindungsgemäß werden so die eingesetzten Disaccharide durch substatspezifische Glycosidasen in die entsprechenden Monosaccharide gespalten. Saccharose wird durch β-Fructofuranosidase (Invertase) (E. C. 3.2.1.26) in Glucose und Fructose, Maltose durch Maltase (E. E. 3.2.1.20) in Glucose; Cellobiose durch Cellobiase (E. C. 3.2.1.21) in Glucose und Lactose durch β-Galactosidase (E. C. 3.2.1.23) in Glucose und Galactose zerlegt. Inulin wird durch Inulinase (E. C. 3.2.1.7) in monomere Fructose gespalten und kann isomerisiert werden.

Wie oben beschrieben, wird die so erhaltene Glucose und/oder Fructose oder direkt zum Einsatz gelangende Monosaccharide werden — falls erforderlich — durch eine glucoseumsetzende Xylose-Isomerase (E. C. 5.3.1.5) in äquimolare Mengen Glucose und Fructose umgewandelt.

Bei dem erfindungsgemäßen Verfahren wird die Glucose durch Glucose-Dehydrogenase zu Gluconsäure autoxidiert, wobei gleichzeitig das verwendete Redoxsystem in seine reduzierte Form überführt wird. Gleichzeitig wird die Fructose durch Sorbit-Dehydrogenase, Mannit-Dehydrogenase oder Polyol-Dehydrogenase zu Sorbit und/oder Mannit reduziert. Dabei wird das beteiligte Redoxsystem wieder in die oxidierte Form überführt (vgl. das oben aufgeführte Reaktionsschema).

Als mögliche Redoxsysteme werden beispielsweise $Cu^{2+}/Cu^{1+}$ oder $Fe^{3+}/Fe^{2+}$ in freier oder anorganisch bzw. organisch komplexierter Form verwendet, z.B. als $K_3Fe(CN)_6/K_4Fe(CN)_6$ oder Cytochrom c. Andererseits können Vitamine wie K3, K2, K1 und Riboflavin oder Chinon/Hydrochinon-Systeme wie z.B. Benzochinon, α- oder β-Naphthochinon, Redoxfarbstoffe wie 2, 6-Dichlorphenol-indophenol, o-Chlorphenolindo-2.6-dichlorphenol, Methylenblau, Wurster's Blau, Triphenyltetrazoliumchlorid und Phenazinmethosulfat oder die Coenzymsysteme $NAD^+/NADH$, $NADP^+/NADPH$, $Q^0$, $Q^2$, $Q^6$, $Q^7$, $FAD/FADH_2$ und FMN verwendet werden.

Besonders bevorzugt ist die Verwendung von Nicotinamid-adenin-dinucleotid ($NAD^+/NADH$) für das beschriebene Verfahren. Das jeweilige zusätzliche Redoxsystem kann entweder in freier gelöster Form eingesetzt werden, bevorzugt ist jedoch bei Verfahren mit Membranreaktoren die Verwendung in polymergebundener gelöster oder enzymgebundener gelöster aber auch in immobilisierter Form. Dadurch wird (ist) sein Molekulargewicht so erhöht, daß es vollständig durch die Membran zurückgehalten wird.

Der abfließende Produktstrom enthält neben Gluconsäure und Sorbit und/oder Mannit je nach Aufbau der Anlage und Aktivität der eingesetzten Enzyme auch nicht umgesetzte Glucose und Fructose. Die Komponenten werden voneinander getrennt durch Adsorptionschromatographie (Aktivkohle- oder Aktivkohle-Celite-Säulen), Verteilungschromatographie (Cellulose- oder Celite-Säulen) oder Ionenaustauschchromatographie.

Die im Produktstrom enthaltene Gluconsäure wird in Form von Gluconsäure-δ-lacton, Natriumgluconat oder Calcium-gluconat gewonnen. Das Gluconsäure-δ-lacton wird durch Auskristallisieren aus der konzentrierten Lösung erhalten. Zur Gewinnung von Natriumgluconat wird die Gluconsäurelösung mit einer äquimolaren Menge Natronlauge versetzt und das dabei gebildete Natriumgluconat durch Trocknen erhalten. Analog wird zur Herstellung von Calciumgluconat die Gluconsäurelösung mit Calciumcarbonat versetzt.

Dem Reaktorinhalt kann enzymatisch inaktives Protein zugegeben werden, um die beteiligten Enzyme vor Inaktivierung zu schützen. Die Menge an zugesetztem Schutzprotein beträgt z.B. im Falle von Albumin das ein- bis das $10^4$-fache der Menge der beteiligten Enzyme. Es sollte nicht mehr als eine 1%ige Schutzproteinlösung resultieren. Aufgrund seines hohen Molekulargewichtes wird auch das Schutzprotein vollständig durch die Membran zurückgehalten.

Die im Reaktor gemeinsam vorliegenden Enzyme haben verschiedene Halbwertszeiten ihrer Inaktivierung. Um über einen längeren Zeitraum hinweg den Reaktor funktionsfähig zu halten, werden die Enzyme dem Reaktor in einem Aktivitätsverhältnis zwischen 10:1 und 1:10 zugefügt, wobei das Enzym mit der schnelleren Inaktivierung anfangs im Überschuß vorliegt. Auch durch Nachdosierung einzelner gelöster Enzyme während des Produktionsprozesses können die Substratumsatzraten je Zeiteinheit von Vorwärts- und Rückwärtsreaktion wieder aufeinander abgestimmt werden.

Einige der benötigten Enzyme sind wie im Falle der Glucose-Dehydrogenase aus Bacillus megaterium (E. C. 1.1.1.47) und Sorbit-Dehydrogenase (E. C. 1.1.1.14) aus Schafsleber im Handel erhältlich. Ebenso ist Xylose-Isomerase (E. C. 5.3.1.5) kommerziell zugänglich (Lectobacillus brevis).

Die weiteren Enzyme lassen sich z.B. aus folgenden Quellen isolieren.

— Sorbit-Dehydrogenase aus Bacillus subtilis (E.C. 1.1.1.14-S.B. Horwitz, Meth. Enzymol. *9*, 155-159 (1966)

— Mannit-Dehydrogenase aus Lactobacillus brevis (E.C. 1.1.1. 67 — B.L. Horecker, Meth. Enzymol. *9*, 143-146 (1966)

— Polyol-Dehydrogenase aus Candida utilis oder Lebergewebe.

(M. Chakravorty, B.L. Horecker, Meth. Enzymol. *9*, 163-166 (1966)

Als Enzymreaktor wird bei dem erfindungsgemäßen Verfahren bevorzugt ein Ultrafiltrationshohlfasermodul eingesetzt. Es ist besonders bevorzugt, vom Material her asymmetrische Hohlfasern aus Polyamid oder Polysulfon mit einer Wandstärke zwischen 30 und 100 µm bei einem Innendurchmesser von 100 bis 400 µm zu verwenden. Es kann auch ein Modul, bei welchem die eigentliche Trennmembran mit z.B. 20.000 Dalton außen sitzt und in dessen Stützmaterial das Enzym-Coenzym-System zusammen mit Schutzprotein eingebracht wird, verwendet werden. Durch einen geeigneten Transmembrandruck wird die kontinuierliche Entfernung des Produkt-Substrat-Gemisches erreicht.

Es ist bevorzugt, daß die eigentliche, für Enzyme und polymergebundene Coenzyme undurchlässige selektive Schicht der Membran auf der Innenseite der Hohlfaser liegt, die enzymatische Reaktion also im Lumen der Hohlfaser stattfindet.

Erfindungsgemäß können bei Molekulargewichten der Produkte von <200 D, auch sehr dünne Membranen verwendet werden, wodurch es möglich wird, ohne Druck zu arbeiten.

Der bei dem erfindungsgemäßen Verfahren verwendete Reaktor kann auf einfache Weise ausgewählt werden, wobei der Reaktor folgende Bedingungen erfüllen muß:

1. Vollständige Rückhaltung der Enzyme, der polymergebundenen Coenzyme (z.B. $NAD^+$/NADH) und des evtl. zugesetzten inerten Schutzproteins,

2. nur minimale Protein- und Coenzymablagerungen auf der Membran, um die Filtrationsleistung langfristig aufrechtzuerhalten.

3. Kontinuierliche Produktabführung, insbesondere von Gluconsäure, um die reaktionsbedingte pH-Absenkung so gering wie möglich zu halten.

Anhand der beigefügten Zeichnungen wird das erfindungsgemäße Verfahren näher erläutert. Es zeigen

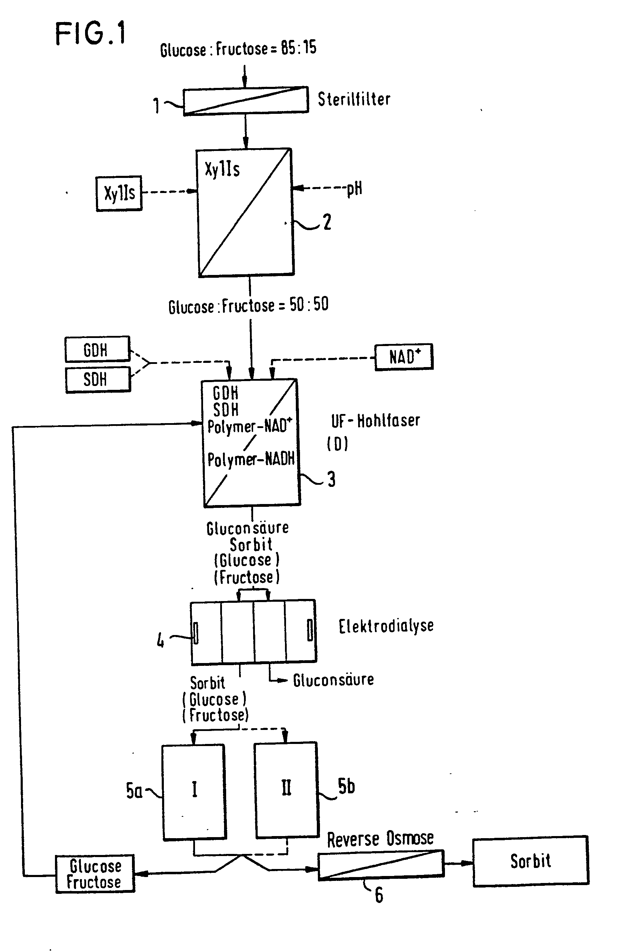

Figur 1: Eine schematische Darstellung des Verfahrens mit einem Hohlfaser-Ultrafiltrationsmodul

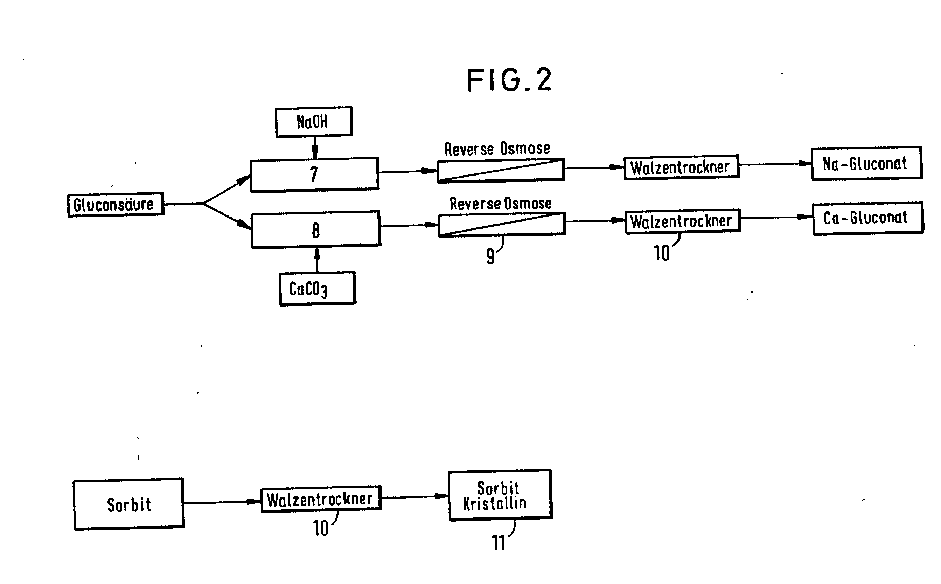

Figur 2: Eine schematische Darstellung der Aufarbeitung von Gluconsäure und Sorbit

Figur 3: Das Prinzip eines Hohlfaser-Ultrafiltrationsmoduls

Figur 4: Den schematischen Aufbau einer für das erfindungsgemäße Verfahren verwendeten Hohlfaser, wobei sich die selektive Schicht auf der Hohlfaseraußenseite befindet.

Figur 5: Eine schematische Darstellung des Verfahrens gemäß Ansprüchen 15, 16 und 17.

Als Ausgangsmaterial wird bevorzugt ein Gemisch verwendet, welches Glucose: Fructose im Verhältnis 85:15 enthält. Das sterilfiltrierte Gemisch 1 wird im Reaktor 2 isomerisiert. Das Glucose/Fructose 1:1 enthaltende Gemisch wird nach Einstellung des pH-Wertes in einen UF-Hohlfaserreaktor 3 (UF = Ultrafiltration) geleitet, wo die eigentliche Reaktion stattfindet. In dem Reaktor befindet sich das Coenzymsystem wie oben aufgeführt, und die Substrate werden in Gluconsäure und Sorbit überführt.

Das abgezogene Ultrafiltrat enthält außer den gewünschten Produkten noch Glucose und Fructose. In einer Elektrodialysestufe 4 erfogt eine Trennung in Gluconsäure und ein Gemisch aus Sorbit sowie restlicher Glucose und Fructose. Das Sorbit wird mit Hilfe eines säulenchromatographischen Verfahrens 5a und 5b, von den nicht umgesetzten Substraten Glucose und Fructose abgetrennt. Stehen mehrere Säulen 5a, 5b zur Verfügung, erfolgt jeweils auf einer Säule die Auftrennung, während die andere(n) gleichzeitig regeneriert wird (werden). Nach der Abtrennung des Sorbits wird das Gemisch aus Glucose und Fructose in den Reaktor 3 zurückgeleitet, wohingegen die Sorbitlösung einer Umkehrosmose 6 unterworfen wird.

In der beigefügten Zeichnung 2 werden die weiteren Aufarbeitungsschritte beispielhaft erläutert.

Die Gluconsäure wird beispielsweise mit Natriumhydroxid 7 oder Calciumcarbonat 8 in Natriumgluconat oder Calciumgluconat überführt. Das weitere Vorgehen erfolgt in an sich bekannter Weise, beispielsweise durch Umkehrosmose 9, durch Trocknen auf einem Walzentrockner 10, Kristallisation 11 etc.

Der Sorbit wird beispielsweise durch Umkehrosmose 9 und anschließend ebenfalls durch Trocknen auf einem Walzentrockner 10 und Kristallisation 11 gereinigt.

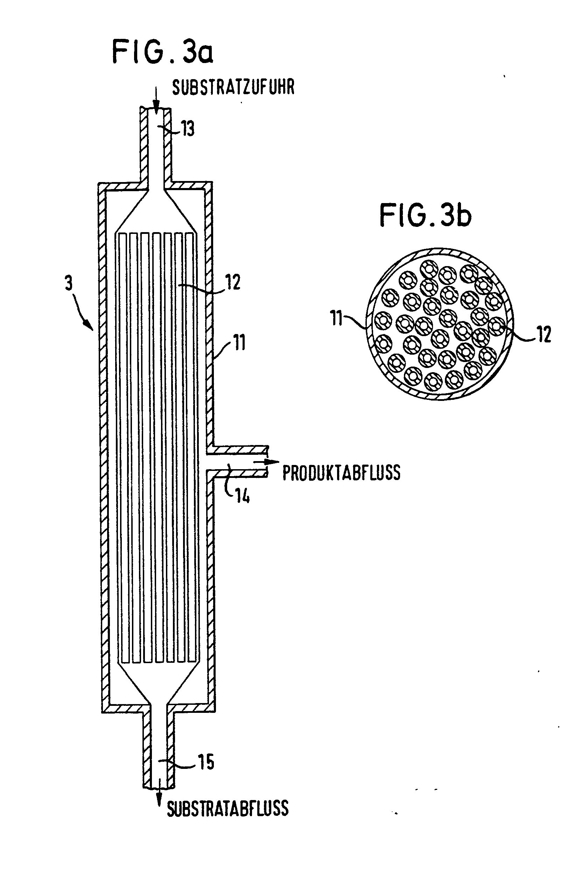

In der beigefügten Figur 3a ist ein Hohlfaser-Ultrafiltrationsmodul 3 im Längsschnitt und in der Figur 3b im Querschnitt dargestellt. Der Ultrafiltrationsreaktor besteht aus einem Gehäuse 11, den Hohlfasern 12, einer Substratzufuhr 13, einem Produktabfluß 14 und einem Substratabfluß 15. In Figur 3b ist der Hohlfaserreaktor im Querschnitt dargestellt. Man erkennt die einzelnen Fasern 12, die einen Innenraum und eine Wand aufweisen.

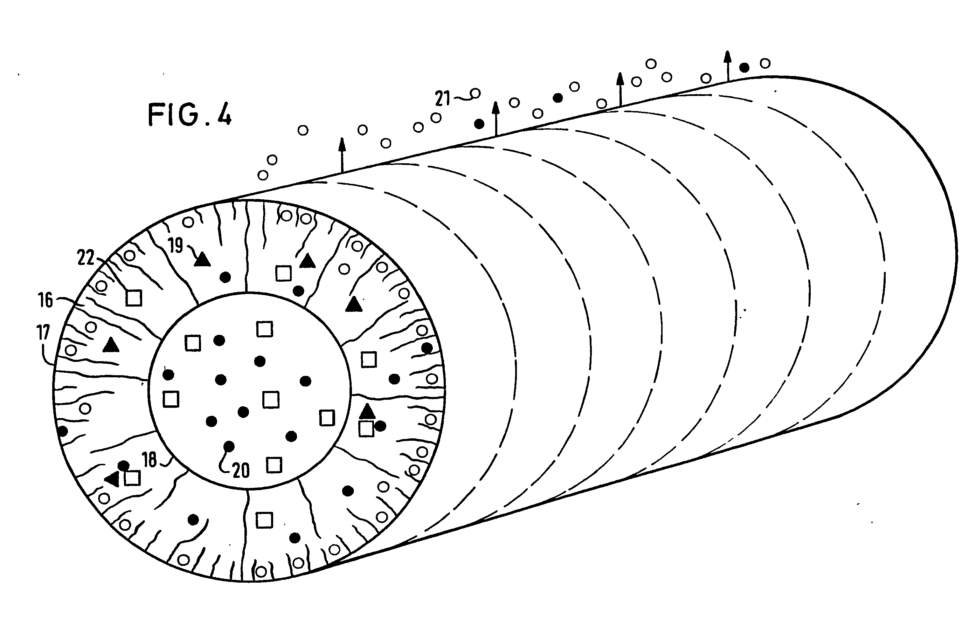

In der Figur 4 ist der schematische Aufbau einer Hohlfaser gemäß Anspruch 7, bei der sich die selektive Schicht auf der Hohlfaseraußenseite befindet, dargestellt. Es bedeuten: 16 die mikroporöse Wand, 17 die semipermeable Membran, die für die Enzyme und Coenzyme durchlässig ist, 18 eine semipermeable Membran, die für das Substrat, das Produkt, die Enzyme und das an das Polymere gebundene Coenzym durchlässig ist, 19 der Biokatalysator (Enzyme), 20 das Substrat, 21 die Produkte und 22 das polymergebundene Coenzym.

Die mikroporöse Wand 16 der Hohlfaser ist auf der Außenseite und der Innenseite jeweils durch eine semipermeable Membran 17, 18 begrenzt. Dabei wird durch den Herstellungsprozeß die äußere semipermeable Membran so eingestellt, daß sie nur für die bei der Reaktion entstehenden Produkte 21 und restliche Substrate 20 durchlässig ist, während die innere Membran sowohl für das Substrat 20, die Enzyme und das polymergebundene Coenzym 22 durchlässig ist. Der Biokatalysator 19 wird in der mikroporösen Schicht zwischen den beiden semipermeablen Membranen adsorptiv immobilisiert. Substrate und Coenzym werden von innen in die Hohlfaser eingeführt. Unter der treibenden Kraft eines Druckes oder Konzentrationsgradienten permeieren Substrat und Coenzym die innere semipermeable Membrane. Sie gelangen in die mikroporöse Wand der Hohlfaser. Dort wird das Substrat mit Hilfe des Biokatalysators in das Produkt umgewandelt, das dann über die äußere Membran die Hohlfaser verläßt.

Um die Hohlfasermembran im technischen Maßstab einsetzen zu können, werden sie als Bündel von einigen tausend Fasern 12 wie in der Abbildung 3 dargestellt, in einem Mantelrohr mit einem Gießharz so eingegossen, daß das Substrat und die Coenzyme an der Stirnseite des Mantelrohres in die Fasern eingeführt werden und das Produkt aus seitlicher Öffnung 14 abgeführt werden kann.

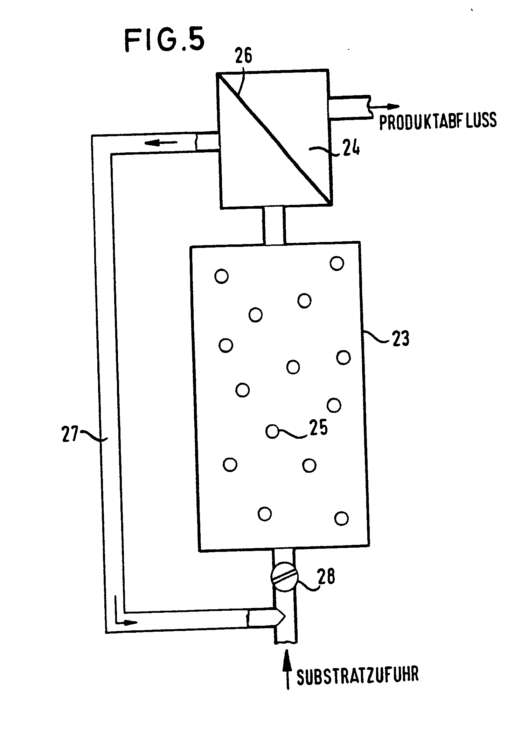

Das erfindungsgemäße Verfahren kann auch in einem Säulenreaktor mit trägerfixierten Enzymen und Coenzymen durchgeführt werden, wobei die Coenzyme auch am gleichen Träger immobilisiert sein können. Das erfindungsgemäße Verfahren kann weiterhin in einem Wirbelbett durchgeführt werden. In Figur 5 ist die Prozeßdurchführung in einem kombinierten Verfahren mit Wirbelbett 23 und Membranmodul 24 schematisch dargestellt. Das Coenzym ist dabei an die Wirbelbettkörper 25 fixiert (adsorptiv oder kovalent) und die Substrate und die Enzyme durchströmen das Wirbelbett 23. Es ist jedoch auch möglich, daß das Enzym oder die Enzyme auf der Oberfläche der Wirbelbettkörper fixiert sind, und daß das Coenzym gelöst, jedoch in polymergebundener Form eingesetzt wird. Das Gemisch aus Restsubstrat, Produkt, Enzym und gegebenenfalls polymergebundenem Coenzym kann dann nach Verlassen des Wirbelbetts durch den Membranmodul 24 geleitet werden, dessen Trennmembran 26 so ausgestattet ist, daß sie nur das Produkt sowie Restsubstrat hindurchlassen, die übrigen Komponenten jedoch aufgrund ihres hohen Molekulargewichts zurückhalten, wobei letztere dem Wirbelbettreaktor über eine Rückführeinrichtung 27 mit Pumpe 28 wieder zugeführt werden.

Die folgenden Beispiele erläutern die Erfindung, ohne sie jedoch zu beschränken.

## Beispiel 1

Enzymatische Herstellung von Calciumgluconat und Sorbit aus Lösungen, die Glucose und Fructose im Verhältnis 1:1 enthalten:

Auf der Membran eines Hohlfaserreaktors mit einer Ausschlußgrenze der Membran von 5 000 D mit 100 cm$^2$ innerer Oberfläche sind je 5 Units der Enzyme Glucose-Dehydrogenase aus Bacillus megaterium und Sorbit-Dehydrogenase aus Schafsleber kovalent immobilisiert. Im Reaktor sind 10$^{-3}$ mol an Polyethylenglycol 6 000 kovalent gebundenes NAD$^+$/NADH eingeschlossen. Der Reaktor wird kontinuierlich von 2,5 ml/h einer wäßrigen, auf pH 6,5 gepufferten Lösung durchflossen, die je 0,1 mol Glucose und Fructose pro Liter gelöst enthält.

Die aus dem Membranreaktor abfließende Lösung enthält neben Gluconsäure und Sorbit auch nicht umgesetzte Glucose und Fructose. Die Produkte werden durch Adsorptionschromatographie an Aktivkohle-Celite-Säulen aufgetrennt. Sorbit wird kristallisiert. Die erhaltene Gluconsäure wird mit einer äquimolaren Menge Calciumcarbonat versetzt, das gebildete Calciumgluconat abfiltriert und getrocknet.

## Beispiel 2

Enzymatische Herstellung von Gluconsäure-δ-lacton und Mannit aus Lösungen, die Glucose und/oder Fructose in beliebigem Verhältnis enthalten:

Zu einem 50 ml fassenden, von einer Ultrafiltrationsmembran mit einem mittleren Porendurchmesser von 1 bis 3 nm verschlossenen Reaktor, der je 500 Units der Enzyme Glucose-Dehydrogenase aus Bacillus megaterium, Mannit-Dehydrogenase aus Lactobacillus brevis und Xylose-Isomerase aus Lactobacillus brevis sowie 1 mmol an wasserlösliches Polyethylenimin mit einem durchschnittlichen Molekulargewicht zwischen 30 000 und 40 000 Daltons kovalent gebundenes NAD$^+$/NADH enthält, wird eine auf pH 6,5 gepufferte Lösung mit insgesamt 180 g/l ≙ 1 mol/l Glucose und Fructose (in beliebigem Verhältnis) kontinuierlich zugeführt, so daß ein Durchsatz von 25 ml/h = 25 mmol/h erreicht wird. Außerdem enthält der Reaktor 0,1% Rinderserumalbumin.

Die abfließende Lösung enthält neben Gluconsäure und Mannit auch nicht umgesetzte Glucose und Fructose. Die Gluconsäure wird mittels Elektrodialyse bei 0,5 bis 0,8 V über eine Anionenaustauschermembran von den restlichen Substanzen abgetrennt. Die gluconsäurehaltige Lösung wird durch Umkehrosmose konzentriert und das sich bildende Gluconsäure-δ-lacton durch Kristallisation gewonnen.

Aus der mannit-, glucose- und fructosehaltigen Lösung wird das Mannit mit Hilfe von Ionenaustauschchromatographie an stark basischen Anionenaustauschern, z.B. Amberlite IRA 400, abgetrennt. Die Fraktionen mit Glucose und Fructose werden in den Prozeß zurückgeführt.

## Beispiel 3

Enzymatische Herstellung von Natriumgluconat, Sorbit und Mannit aus saccharosehaltigen Lösungen:

Eine auf pH 6 — 7 gepufferte Lösung, die 360 g/l ≙ 1 mol/l Saccharose enthält, wird mit einer Geschwindigkeit von 10 ml/min über eine Säule von 8 cm Länge und 2 cm innerem Durchmesser geleitet, die 12 000 Units (≙ 120% der theoretisch benötigten Menge) kovalent an Glasperlen gebundene β-Fructofuranosidase (Invertase) enthält. Die abfließende Lösung wird über eine zweite Säule mit gleichen Dimensionen geführt, die mit Polyacrylamid gefüllt ist, an dem je 12 000 Units (≙ 120% der theoretisch benötigten Menge) Glucose-Dehydrogenase aus Bacillus megaterium und Polyol-Dehydrogenase aus Candida utilis und 16 mmol NAD$^+$ mit einer Restaktivität von mindestens 75% fixiert sind.

Die aus der zweiten Säule ausfließende Lösung enthält Gluconsäure, Mannit und Sorbit, außerdem noch geringe Mengen Glucose und Fructose. Die Substanzen werden mittels Verteilungschromatographie an Celite-Säulen getrennt und die Glucose und Fructose wieder in die zweite Säule eingespeist. Die

# EP 0 132 557 B1

gluconsäurehaltige Fraktion wird mit einer äquimolaren Menge Natronlauge versetzt und das gebildete Natriumgluconat getrocknet.

## Patentansprüche

1. Verfahren zur kontinuierlichen enzymatischen Herstellung von Gluconsäure und/oder ihren Derivaten und
   i. Sorbit oder
   ii. Mannit oder
   iii. Sorbit und Mannit
   aus Glucose- und/oder Fructose enthaltenden Disacchariden, dadurch gekennzeichnet, daß man das als Ausgangssubstrat vorliegende Disaccharid in einem mit dem Redox-Vorgang gleichzeitig ablaufenden oder vorgeschalteten Reaktionsschritt durch spezifische Glykosidasen in die entsprechenden Monosaccharide spaltet und daß man gegebenenfalls ein Glucose-Fructose-Verhältnis von ca. 1:1 durch ebenfalls gleichzeitige oder vorgeschaltete enzymatische Isomerisierung mittels glucoseumsetzender Xylose-Isomerase oder durch chemische Isomerisierung einstellt und
   in einen Reaktor, der Glucose-Dehydrogenase und
   i. Sorbit-Dehydrogenase oder
   ii. Mannit-Dehydrogenase oder
   iii. Polyol-Dehydrogenase sowie gegebenenfalls noch weitere Dehydrogenasen, Isomerasen oder spezifische Glykosidasen und ein
   zusätzliches Redoxsystem enthält, kontinuierlich eine wäßrige Lösung von Glucose und/oder Fructose oder eine wäßrige Lösung von einem Glucose und/oder Fructose enthaltenden Disaccharid zuführt und aus dem Reaktor kontinuierlich eine wäßrige Lösung mit den gebildeten Produkten abführt.

2. Verfahren nach Anspruch 1, dadurch gekennzeichnet, daß man Glucose-Dehydrogenase und Sorbit-Dehydrogenase oder Mannit-Dehydrogenase oder Polyol-Dehydrogenase in einem Aktivitätsverhältnis zwischen 10:1 und 1:10 einsetzt.

3. Verfahren nach Anspruch 1, dadurch gekennzeichnet, daß bei der Oxidations-Reduktionsreaktion der pH-Wert zwischen 5 und 9, vorzugsweise zwischen 6 und 8 gehalten wird.

4. Verfahren nach einem der Ansprüche 1, 2 oder 3, dadurch gekennzeichnet, daß man als zusätzliches Redoxsystem $Cu^{2+}/Cu^{1+}$ oder $Fe^{3+}/Fe^{2+}$-Komplexe, wie $K_3Fe(CN)_6/K_4Fe(CN)_6$, Cytochrom c, Vitamine wie K3, K2 oder K1, Riboflavin, Benzochinon/Hydrochinon, α-Naphthochinon, β-Naphthochinon, Redoxfarbstoffe wie 2.6-Dichlor-phenol-indophenol, o-Chlorphenolindo-2.6-dichlorphenol, Methylenblau, Wurster's Blau, Triphenyltetrazoliumchlorid, Phenazinmethosulfat oder Coenzymsysteme wie $NAD^+/NADH$, $NADP^+/NADPH$, $Q^0$, $Q^2$, $Q^6$, $Q^7$, FAD/FADH, FMN in freier, polymer- oder enzymgebundener Form gelöst oder immobilisiert verwendet, bevorzugt jedoch das Nicotinamid-adenin-dinucleotid ($NAD^+/NADH$) einsetzt.

5. Verfahren nach mindestens einem der vorhergehenden Ansprüche, dadurch gekennzeichnet, daß man die Oxidations-Reduktionsreaktionen in einem Reaktor mit Ultrafiltrationsmembran durchführt, ein Coenzymsystem verwendet, das an ein wasserlösliches Polymer mit einem durchschnittlichen Molekulargewicht zwischen 5.000 und 60.000 Dalton gebunden ist, über die Membran eine Druckdifferenz erzeugt und hinter der Membran einen kontinuierlichen Produktstrom abführt.

6. Verfahren nach mindestens einem der vorhergehenden Ansprüche, dadurch gekennzeichnet, daß man als Membranen hohle Fasern verwendet, die in ihrer Struktur asymmetrisch aufgebaut sind, wobei die für Enzyme und polymergebundene Coenzyme undurchlässige selektive Schicht der Membran auf der Außenseite der Hohlfaser liegt, während die Innenseite der Faser Poren enthält, die auch für die Enzyme und Coenzyme durchlässig sind.

7. Verfahren nach Anspruch 6, dadurch gekennzeichnet, daß man die Substratlösung auf der Innenseite der Hohlfasermembran einführt, zwischen der Innenwand und der Außenwand der Faser eine hydrostatische Druckdifferenz erzeugt, so daß die Substratlösung durch die innere Faserwand hindurch geht und daß die enzymatische(n) Reaktion(en) bevorzugt im porösen Stützmaterial zwischen Innenwand und Außenwand abläuft (ablaufen).

8. Verfahren nach einem der Ansprüche 1, 5, 6 oder 7, dadurch gekennzeichnet, daß man den Durchsatz des Substrats durch geeignete Wahl der hydrostatischen Druckdifferenz so einstellt, daß seine Aufenthaltszeit in der Hohlfaserwand ausreicht, um eine möglichst vollständige Umsetzung zu erzielen.

9. Verfahren nach Anspruch 6, dadurch gekennzeichnet, daß die eigentliche für Enzyme und polymergebundene Coenzyme undurchlässige selektive Schicht der Membran auf der Innenseite der Hohlfaser liegt, die enzymatische Reaktion also im Lumen der Hohlfaser stattfindet.

10. Verfahren nach mindestens einem der Ansprüche 1 bis 9, dadurch gekennzeichnet, daß man zu der Reaktionslösung ein Schutzprotein in einer Menge, die dem 1- bis $10^4$-fachen der Menge der beteiligten Enzyme entspricht, zusetzt.

11. Verfahren nach mindestens einem der Ansprüche 1 bis 10, dadurch gekennzeichnet, daß die Enzyme oder Coenzyme kovalent an der Membran des Reaktors immobilisiert sind.

12. Verfahren nach mindestens einem der vorhergehenden Ansprüche, dadurch gekennzeichnet, daß die Coenzyme an wasserlösliche Polymere gebunden sind.

13. Verfahren nach einem der Ansprüche 1 bis 4, dadurch gekennzeichnet, daß die Oxidations-Reduktionsreaktionen in einem Säulenreaktor mit trägerfixierten Enzymen und Coenzymen durchgeführt werden, wobei die Coenzyme auch gleichen Träger immobilisiert sein können.

14. Verfahren nach einem der Ansprüche 1 bis 4 oder 10, dadurch gekennzeichnet, daß die Oxidations-Reduktionsreaktionen in einem Wirbelbett durchgeführt werden, wobei das Coenzym auf der Oberfläche der Wirbelbett-Körper fixiert ist und das Substrat plus Enzym — das Wirbelbett durchströmt.

15. Verfahren nach einem der Ansprüche 1 bis 4 oder 10, dadurch gekennzeichnet, daß das Enzym oder die Enzyme auf der Oberfläche der Wirbelbett-Körper fixiert ist oder sind und daß das Coenzym gelöst, jedoch in polymergebundener Form eingesetzt wird.

16. Verfahren nach einem der Ansprüche 1 bis 4 oder 14, dadurch gekennzeichnet, daß das Gemisch aus Restsubstrat, Produkt und Enzym oder polymergebundenen Coenzymen nach Verlassen des Wirbelbettes einen Membranmodul durchströmt, dessen Membranporen so ausgestaltet sind, daß sie nur das Produkt sowie Restsubstrat hindurchlassen, die übrigen Komponenten jedoch zurückhalten, wobei letztere dem Wirbelbett-Reaktor wieder zugeführt werden.

17. Verfahren nach mindestens einem der vorhergehenden Ansprüche, dadurch gekennzeichnet, daß man die gebildeten Produkte Gluconsäure oder ihre Derivate und Sorbit oder Mannit oder Sorbit und Mannit durch
(1) Elektrodialyse,
(2) Ionenaustausch-Trennprozesse,
(3) andere Membranverfahren,
(4) eine Kombination der o.g. Verfahren
in Gruppen gleichen Ladungszustands trennt und dabei teilweise konzentriert.

18. Verfahren nach mindestens einem der vorhergenden Ansprüche, dadurch gekennzeichnet, daß man die Zuckeralkohole Sorbit und/oder Mannit durch
(1) Adsorptionschromatographie,
(2) Verteilungschromatographie,
(3) Ionenaustauschchromatographie,
(4) fraktionierte Kristallisation
von den Resten an Glucose und Fructose abtrennt und separat weiterarbeitet.

19. Vorrichtung zur Durchführung des Verfahrens nach einem der vorhergehenden Ansprüche, gekennzeichnet durch zwei hintereinandergeschaltete UF-Hohlfaserreaktoren (2, 3), wobei im ersten die Isomerisierungsreaktion, im zweiten die Redoxreaktionen ablaufen, sowie Trenn- und Reinigungsvorrichtungen (4, 5a, 5b, 6) und übliche Zu- und Ableitungsfilter, Regeleinrichtungen und Probeentnahmeeinrichtungen.

**Revendications**

1. Procédé de préparation enzymatique en continu d'acide gluconique et/ou de ses dérivés et
a) de sorbitol ou
b) de mannitol ou
c) de sorbitol et de mannitol
à partir de disaccharides contenant du glucose et/ou du fructose, caractérisé en ce qu'on sépare le saccharide servant de substrat de départ en monosaccharides correspondants au moyen de glucosidases spécifiques au cours d'une phase réactionnelle se déroulant pendant ou avant l'opération d'oxydoréduction et que l'on ajuste, le cas échéant, un rapport glucose-fructose d'environ 1:1 par isomérisation enzymatique également simultanée ou préalable au moyen d'une xylose-isomérase transformant le glucose ou par isomérisation chimique,
et dans un réacteur qui contient de la glucose-déshydrogénase et
a) de la sorbitol-déshydrogénase ou
b) de la mannitol-déshydrogénase ou
c) de la polyol-déshydrogénase ainsi que, le cas échéant, d'autres déshydrogénases, isomérases ou glucosidases spécifiques et
un système d'oxydoréduction supplémentaire, on introduit en continu une solution aqueuse de glucose et/ou de fructose ou une solution aqueuse d'un disaccharide contenant du glucose et/ou du fructose et on soutire en continu du réacteur une solution aqueuse contenant les produits formés.

2. Procédé selon la revendication 1, caractérisé en ce qu'on met en oeuvre la glucose-déshydrogénase et la sorbitol-déshydrogénase ou la mannitol-déshydrogénase ou la polyol-déshydrogénase selon un rapport d'activité compris entre 10:1 et 1:10.

3. Procédé selon la revendication 1, caractérisé en qu'on maintient le pH, pendant la réaction d'oxydoréduction, à une valeur comprise entre 5 et 9, de préférence entre 6 et 8.

4. Procédé selon l'une des revendications 1, 2 et 3, caractérisé en ce qu'on met en oeuvre comme système d'oxydoréduction supplémentaire des complexes $Cu^{2+}/Cu^{1+}$ ou $Fe^{3+}/Fe^{2+}$, tels que $K_3Fe(CN)_6/K_4(CN)_6$, le cytochrome c, des vitamines telles que la K3, la K2 ou la K1, la riboflavine, la benzoquinone-hydroquinone, l'α-naphtoquinone, la β-naphtoquinone, des colorants d'oxydoréduction tels que le 2,6-dichloro-phénol-indophénol, l'o-chlorophénol-indo-2,6-dichlorophénol, le bleu de méthylène, le bleu de Wurster, le chlorure de triphényltétrazolium, le méthosulfate de phénazine ou des coenzymes telles que $NAD^+$/NADH, $NADP^+$/ NADPH, $Q^0$, $Q^2$, $Q^6$, $Q^7$, FAD/FADH, FMN sous une forme libre, liée au polymère ou à l'enzyme, dissoutes ou immobilisées, mais on met en oeuvre de préférence le nicotineamide-adénine-dinucléotide ($NAD^+$/NADH).

5. Procédé selon au moins l'une des revendications précédentes, caractérisé en ce qu'on réalise les réactions d'oxydoréduction dans un réacteur comportant une membrane d'ultrafiltration, on met en oeuvre un système de coenzymes qui est lié à un polymère soluble dans l'eau et ayant un poids moléculaire moyen compris entre 5.000 et 60.000 daltons, on crée une différence de pression au moyen de la membrane et on soutire un flux continu de produit derrière la membrane.

6. Procédé selon au moins l'une des revendications précédentes, caractérisé en ce qu'on utilise comme membranes des fibres creuses qui présentent une structure asymétrique, dans lequel la couche sélective de la membrane, qui ne laisse pas passer les enzymes et les coenzymes liées au polymère, est disposée sur le côté extérieur de la fibre creuse tandis que le côté intérieur de la fibre contient des pores qui peuvent être traversés aussi par les enzymes et les coenzymes.

7. Procédé selon la revendication 6, caractérisé en ce qu'on introduit la solution de substrat par le côté intérieur de la membrane en fibre creuse, on crée une différence de pression hydrostatique entre la paroi intérieure et la paroi extérieure de la fibre de sorte que la solution de substrat traverse la paroi intérieure de la fibre et que la/les réaction(s) enzymatique(s) se déroule(nt) de préférence dans le matériau poreux de support entre la paroi intérieure et la paroi extérieure.

8. Procédé selon l'une des revendications 1, 5, 6 ou 7, caractérisé en ce qu'on règle le débit du substrat, par un choix approprié de la différence de pression hydrostatique, de manière telle que sa durée de séjour dans la paroi de fibre creuse soit suffisante pour obtenir une transformation aussi complète que possible.

9. Procédé selon la revendication 6, caractérisé en ce que la couche sélective proprement dite de la membrane, qui ne laisse pas passer les enzymes et les coenzymes liées au polymère, est disposée sur le côté intérieur de la fibre creuse, la réaction enzymatique s'effectuant donc dans le lumen de la fibre creuse.

10. Procédé selon au moins l'une des revendications 1 à 9, caractérisé en ce qu'on ajoute à la solution réactionnelle une protéine protectrice selon une quantité qui équivaut à 1 fois jusqu'à $10^4$ fois la quantité des enzymes participant à la réaction.

11. Procédé selon au moins l'une des revendications 1 à 10, caractérisé en ce que les enzymes ou coenzymes sont immobilisées de manière covalente sur la membrane du réacteur.

12. Procédé selon au moins l'une des revendications précédentes, caractérisé en ce que les coenzymes sont liées à des polymères solubles dans l'eau.

13. Procédé selon l'une des revendications 1 à 4, caractérisé en ce que les réactions d'oxydoréduction sont réalisées dans un réacteur à colonne avec des enzymes et des coenzymes fixées à un support, les coenzymes pouvant aussi être immobilisées sur le même support.

14. Procédé selon l'une des revendications 1 à 4 ou selon la revendication 10, caractérisé en ce que les réactions d'oxydoréduction sont réalisés dans un lit tourbillonnaire, la coenzyme étant fixée à la surface du corps de lit tourbillonnaire et le substrat plus l'enzyme traversant le lit tourbillonnaire.

15. Procédé selon l'une des revendications 1 à 4 ou selon la revendication 10, caractérisé en ce que l'enzyme ou les enzymes est/sont fixée(s) à la surface du corps de lit tourbillonnaire et que la coenzyme est mise en oeuvre dissoute, mais sous une forme liée au polymère.

16. Procédé selon l'une des revendications 1 à 4 ou selon la revendication 14, caractérisé en ce que le mélange composé du substrat résiduel, du produit et de l'enzyme ou des coenzymes liées au polymère, après avoir quitté le lit tourbillonnaire, traverse un module à membrane dont les pores sont conformés de manière à ne laisser passer que le produit ainsi que le substrat résiduel mais ils retiennent les autres composants, ces derniers étant réintroduits dans le réacteur à lit tourbillonnaire.

17. Procédé selon au moins l'une des revendications précédentes, caractérisé en ce qu'on sépare les produits formés de l'acide gluconique ou ses dérivés et le sorbitol ou le mannitol ou le sorbitol et le mannitol par
(1) électrodialyse,
(2) des procédés de séparation par échange d'ions,
(3) d'autres procédés à membrane,
(4) une combinaison des procédés cités ci-dessus
en groupes ayant le même état de charge et, de ce fait, on les concentre partiellement.

18. Procédé selon au moins l'une des revendications précédentes, caractérisé en ce qu'on isole les alcools dérivés du sucre, c'est-à-dire les sorbitol et/ou mannitol par cools de sucre sorbitol et/ou mannitol par
(1) chromatographie par adsorption,
(2) chromatographie de répartition,
(3) chromatographie à échange d'ions,
(4) cristallisation fractionnée
des résidus en glucose et en fructose et on les traite séparément.

19. Dispositif pour la mise en oeuvre du procédé selon l'une des revendications précédentes, caractérisé par deux réacteurs à ultra-fréquence à fibre creuse (2, 3), la réaction d'isomérisation s'effectuant dans le premier réacteur et les réactions d'oxydoréduction s'effectuant dans le second réacteur, et par des

# EP 0 132 557 B1

dispositifs de séparation et de purification (4, 5a, 5b, 6) et des filtres usuels d'introduction et d'évacuation, des dispositifs de régulation et des dispositifs de prélèvement d'échantillons.

## Claims

1. A method of continuous enzymatic production of gluconic acid and/or derivatives thereof and
   i. Sorbitol or
   ii. Mannitol or
   iii. Sorbitol and mannitol
   from disaccharides containing glucose and/or fructose, characterised in that the disaccharide starting substrate is split into the corresponding monosaccharides by specific glycosidases in a reaction step occurring simultaneously or before the redox process, and if required the glucose/fructose ratio is adjusted to about 1:1 by enzymatic isomerization (likewise simultaneous or previous) by glucose-converting xylose isomerase or by chemical isomerization, and
   an aqueous solution of glucose and/or fructose or an aqueous solution of a disaccharide containing glucose and/or fructose is continuously supplied to a reactor containing glucose dehydrogenase and
   i. Sorbitol dehydrogenase or
   ii. Mannitol dehydrogenase or
   iii. Polyol dehydrogenase and, if required, additional dehydrogenases, isomerases or specific glycosidases and an additional redox system, and
   an aqueous solution containing the products formed is continuously withdrawn from the reactor.

2. A method according to claim 1, characterised in that glucose dehydrogenase and sorbitol dehydrogenase or mannitol dehydrogenase or polyol dehydrogenase are used in an activity ratio between 10:1 and 1:10.

3. A method according to claim 1, characterised in that in the oxidation-reduction reaction the pH is kept between 5 and 9, preferably between 6 and 8.

4. A method according to any of claims 1, 2 or 3, characterised by use of the following substances as the additional redox system: $Cu^{2+}/Cu^{1+}$ or $Fe^{3+}/Fe^{2+}$ complexes such as $K_3Fe(CN)_6/K_4Fe(CN)_6$, cytochrome c, vitamins such as K3, K2 or K1, riboflavin, benzoquinone/hydroquinone, α-naphthoquinone, β-naphthoquinone, reflex dyes such as 2.6-dichloro-phenol-indophenol, o-chlorophenolindo-2.6-dichlorophenol, methylene blue, Wurster's blue, triphenyl tetrazolium chloride, phenazine methosulphate or coenzyme systems such as $NAD^+/NADH$, $NADP^+/NADPH$, $Q^0$, $Q^2$, $Q^6$, $Q^7$, FAD/FADH, FMN dissolved or immobilised in free polymeric or enzyme-bonded form, but preferably use is made of nicotineamide adenine dinucleotide ($NAD^+/NADH$).

5. A method according to at least one of the preceding claims, characterised in that the oxidation-reduction reactions are carried out in a reactor comprising an ultrafiltration membrane, use is made of a coenzyme system bonded to a water-soluble polymer having an average molecular weight between 5000 and 60000 Daltons, a pressure difference is produced through the membrane, and a continuous stream of product is withdrawn behind the membrane.

6. A method according to at least one of the preceding claims, characterised in that hollow fibres having an asymmetrical structure are used as membranes, the selective layer of the membrane impermeable to enzymes and polymer-bonded coenzymes being on the outside of the hollow fibre whereas the inner side of the fibre has pores which are also permeable to enzymes and coenzymes.

7. A method according to claim 6, characterised in that the subtrate solution is introduced on the inner side of the hollow-fibre membrane, a hydrostatic pressure difference is produced between the inner wall and the outer wall of the fibre so that the substrate solution goes through the inner fibre wall, and the enzymatic reaction or reactions are preferably carried out in the porous supporting material between the inner wall and the outer wall.

8. A method according to any of claims 1, 5, 6 or 7, characterised in that the throughput of substrate is adjusted, by suitably choosing the hydrostatic pressure difference, so that its residence time in the hollow fibre wall is sufficient to obtain substantially complete conversion.

9. A method according to claim 6, characterised in that the actual selective layer of the membrane which is impermeable to enzymes and polymer-bonded coenzymes is disposed on the inner side of the hollow fibre, i.e. the enzymatic reaction occurs in the lumen of the hollow fibre.

10. A method according to at least one of claims 1 to 9, characterised in that a protective protein is added to the reaction solution in a quantity equal to 1 to $10^4$ times the quantity of enzyme involved.

11. A method according to at least one of claims 1 to 10, characterised in that the enzymes or coenzymes are covalently immobilised on the reactor membrane.

12. A method according to at least one of the preceding claims, characterised in that the coenzymes are bonded to water-soluble polymers.

13. A method according to any of claims 1 to 4, characterised in that the oxidation-reduction reactions are carried out in a column reactor comprising enzymes and coenzymes fixed to a support, the coenzymes also optionally being immobilised on the same support.

14. A method according to any of claims 1 to 4, or 10, characterised in that the oxidation-reduction reactions are carried out in a fluidized bed, the coenzyme being fixed on the surface of the fluidized-bed member and the substrate and enzyme flowing through the fluidized bed.

15. A method according to any of claims 1 to 4 or 10, characterised in that the enzyme or enzymes are fixed to the surface of the fluidized-bed member and the coenzyme is used in dissolved but polymer-bonded form.

16. A method according to any of claims 1 to 4 or 14, characterised in that the mixture of residual substrate, product and enzyme or polymer-bonded coenzymes, after leaving the fluidized-bed, flows through a membrane module having pores shaped so that they are permeable to the product or residual substrate only but hold back the other components, which are returned to the fluidized-bed reactor.

17. A method according to at least one of the preceding claims, characterised in that the products, gluconic acid or derivatives thereof, and sorbitol or mannitol or sorbitol and mannitol are separated into similarly-charged groups and, in the process, partially concentrated by

(1) Electrodialysis,
(2) Ion-exchange separation processes,
(3) Other membrane processes or
(4) A combination of the aforementioned processes.

18. A method according to at least one of the preceding claims, characterised in that the sugar alcohols sorbitol and/or mannitol are separated from the residual glucose and fructose and separately further processed by

(1) Adsorption chromatography,
(2) Distribution chromatography,
(3) Ion-exchange chromatography or
(4) Fractional crystallization.

19. A device for working the method according to any of the preceding claims, characterised by two UF hollow-fibre reactors (2, 3) in series, the isomerization reaction occurring in the first and the redox reactions occurring in the second, and separating and purifying devices (4, 5a, 5b, 6) and conventional supply and discharge filters, control devices and sample-taking devices.

# FIG.1

FIG.2

EP 0 132 557 B1

# FIG. 3a

SUBSTRATZUFUHR

13

3

12

11

PRODUKTABFLUSS

14

15

SUBSTRATABFLUSS

# FIG. 3b

11

12

FIG. 4

# FIG.5

PRODUKTABFLUSS

SUBSTRATZUFUHR